# EUROPEAN PATENT APPLICATION

(11) **EP 4 286 845 A2**
(43) Date of publication of application: **06.12.2023**
(21) Application number: 23175703.0
(22) Date of filing: 26.05.2023
(51) Int. Cl.: G01N 33/00

(54) **GAS DETECTION DEVICE AND GAS DETECTION SYSTEM**

(30) Priority: 31.05.2022 JP 2022088789
(71) Applicant: Ricoh Company, Ltd., Tokyo 143-8555 (JP)
(72) Inventor: UJIMOTO, Katsuya, Tokyo, 143-8555 (JP); TAN, Kunihiro, Tokyo, 143-8555 (JP); KUBOTA, Shinichi, Toyko, 143-8555 (JP)
(74) Representative: SSM Sandmair

(57) **Abstract**

A gas detection device (500) includes a first detector (100) to output gas information being information on a gas sucked from each of a plurality of inlets (21) arranged at different positions, a second detector (200) to output object position information indicating a position of an object, and a processor (300; 300a). Based on gas spatial distribution information based on the gas information from the first detector (100), the object position information from the second detector (200), and first relative position information indicating a position of each of the plurality of inlets relative to the object, the processor (300; 300a) outputs second relative position information indicating gas spatial distribution relative to the position of the object.

## Description

### BACKGROUND

### Technical Field

The present disclosure relates to a gas detection device and a gas detection system.

### Related Art

Gas detection devices to detect the type, odor, or the like of gas have been developed. The gas detection device is used, for example, in the evaluation of the maturation of fruit in agriculture, the evaluation of food freshness in food retailing, the detection of a strange odor in a factory, and health management based on halitosis in medical treatment.

For example, Japanese Patent No. 6655597 discloses a gas detection device that uses a pattern included in spectrum data output from at least one sensor to detect a chemical substance and image information obtained by capturing an object, in order to recognize the obj ect.

There is a demand for a gas detection device capable of outputting spatial distribution information of gas.

### SUMMARY

In one aspect, a gas detection device includes a first detector to output gas information being information on a gas sucked from each of a plurality of inlets arranged at different positions, a second detector to output object position information indicating a position of an object, and a processor. Based on gas spatial distribution information based on the gas information from the first detector, the object position information from the second detector, and first relative position information indicating a position of each of the plurality of inlets relative to the object, the processor outputs second relative position information indicating gas spatial distribution relative to the position of the object.

In another aspect, a gas detection system includes a traveling body including a first detector to output gas information being information on inhaled gas, a position information acquisition unit to output position information indicating a position of the first detector moved by the traveling body, and a processor. The processor outputs gas spatial distribution information based on the gas information from the first detector and the position information from the position information acquisition unit.

According to the aspects of the present disclosure, the gas detection device and the gas detection system capable of outputting spatial distribution information of gas are provided.

### BRIEF DESCRIPTION OF THE DRAWINGS

A more complete appreciation of embodiments of the present disclosure and many of the attendant advantages and features thereof can be readily obtained and understood from the following detailed description with reference to the accompanying drawings, wherein:
FIG. 1 is a block diagram illustrating the overall configuration of a gas detection device according to embodiments;
FIG. 2 is a block diagram illustrating a functional configuration of a processor according to a first embodiment;
FIG. 3 is a diagram illustrating object information according to a first embodiment;
FIG. 4 is a diagram illustrating first relative position information according to the first embodiment;
FIG. 5 is a diagram illustrating second relative position information according to the first embodiment;
FIG. 6 is a diagram illustrating a configuration of a first detector according to the first embodiment;
FIG. 7 is a block diagram illustrating a functional configuration of the first detector according to the first embodiment;
FIG. 8 is a perspective view illustrating a configuration of a switching unit of the first detector according to the first embodiment;
FIG. 9 is a top view of the switching unit of the first detector illustrated in FIG. 8;
FIG. 10 is a cross-sectional view taken along a line V-V in FIG. 9;
FIG. 11 is a cross-sectional view taken along a line VI-VI in FIG. 9;
FIG. 12 is a block diagram illustrating a functional configuration of a controller of the first detector according to the first embodiment;
FIG. 13 is a flowchart illustrating an operation of the first detector according to the first embodiment;
FIG. 14 is a block diagram illustrating a functional configuration of a processor according to a second embodiment;
FIG. 15 is a diagram illustrating a configuration of a gas detection system according to a third embodiment;
FIG. 16 is a block diagram illustrating a functional configuration of a processor according to a third embodiment;
FIG. 17 is a diagram illustrating gas spatial distribution information according to the

### third embodiment; and

FIGS. 18A to 18D are diagrams illustrating captured images of a mountain road, urban areas, and an expressway according to the third embodiment.

The accompanying drawings are intended to depict embodiments of the present disclosure and should not be interpreted to limit the scope thereof. The accompanying drawings are not to be considered as drawn to scale unless explicitly noted. Also, identical or similar reference numerals designate identical or similar components throughout the several views.

### DETAILED DESCRIPTION

In describing embodiments illustrated in the drawings, specific terminology is employed for the sake of clarity. However, the disclosure of this specification is not intended to be limited to the specific terminology so selected and it is to be understood that each specific element includes all technical equivalents that have a similar function, operate in a similar manner, and achieve a similar result.

Referring now to the drawings, embodiments of the present disclosure are described below. As used herein, the singular forms "a," "an," and "the" are intended to include the plural forms as well, unless the context clearly indicates otherwise.

Referring to the drawings, a gas detection device and a gas detection system according to embodiments of the present disclosure are described. However, the embodiments described below are some examples of the gas detection device and the gas detection system for embodying the technical idea of the present disclosure, and embodiments of the present disclosure are not limited to the examples described below. The dimensions, materials, and shapes of components, relative arrangements thereof, and the like described below are not intended to limit the scope of the present disclosure unless otherwise specified and are only examples for explanation. The size, positional relation, and the like of components illustrated in the drawings may be exaggerated for use of description. In the following description, the same or equivalent components are given the same or similar name and reference signs, and redundant descriptions are omitted as appropriate.

### Embodiments

### Overall Configuration of Gas Detection Device

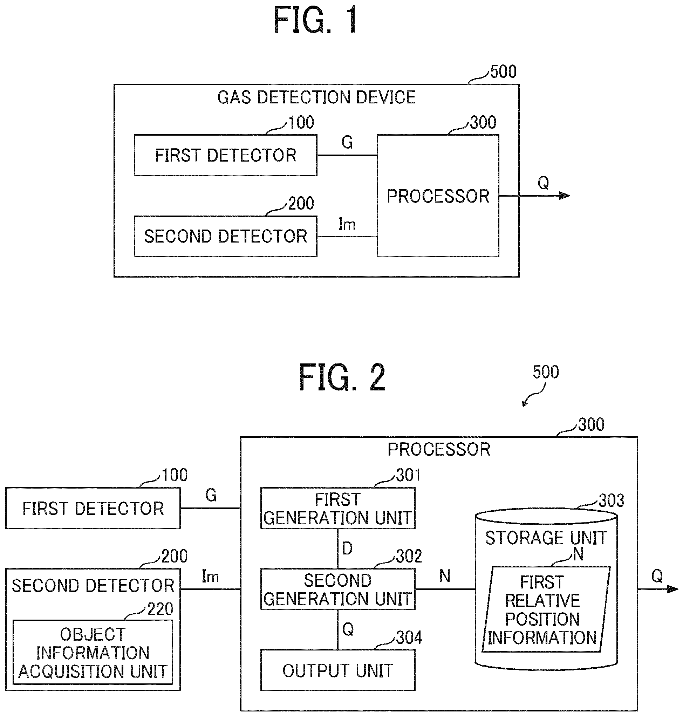

FIG. 1 is a block diagram illustrating a general arrangement of a gas detection device 500 according to embodiments.

The gas detection device 500 includes a first detector 100, a second detector 200, and a processor 300.

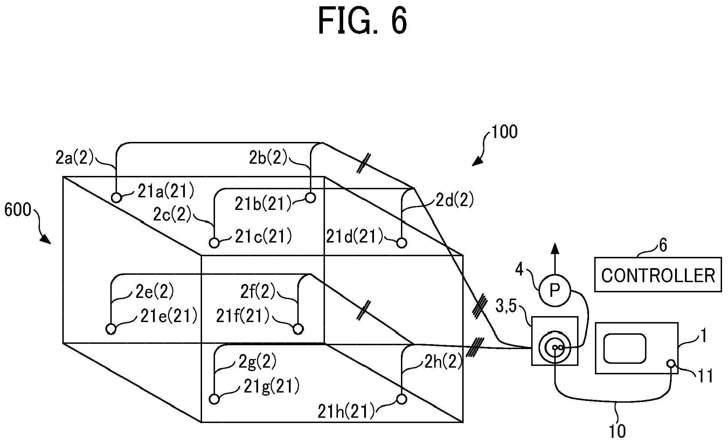

The first detector 100 outputs, as gas information G, information on gas sucked from each of a plurality of inlets arranged at different positions. The configuration, function, and operation of the first detector 100 will be described later with reference to FIGS. 6 to 13.

The second detector 200 outputs object position information Im that is information indicating positions of objects. In the present embodiment, the second detector 200 includes an object information acquisition device 220 that is an image capturing device to output a captured image of an object. The image capturing device is a camera including an imaging element such as a charge coupled device (CCD) or a complementary metal oxide semiconductor (CMOS), and a lens that forms an image of an object (in other words, a subject) on an imaging surface of the imaging element. The image capturing device may be a wide-angle camera capable of performing wide-angle imaging or a spherical camera (omnidirectional camera) capable of performing imaging in all directions including up, down, left, and right of the camera at a time, to acquire object information in a wide spatial range at a time.

The processor 300 includes, for example, an information processing device such as a personal computer (PC). The processor 300 outputs second relative position information Q indicating gas spatial distribution relative to the position of an object based on gas spatial distribution information D based on the gas information G from the first detector 100, the object position information Im from the second detector 200, and first relative position information N indicating a relative position of each of the plurality of inlets relative to the object.

### First Embodiment

### Functional Configuration of Processor 300

FIG. 2 is a block diagram illustrating a functional configuration of the processor 300 of the gas detection device 500 according to a first embodiment. The processor 300 includes a first generation unit 301, a second generation unit 302, a storage unit 303, and an output unit 304.

The processor 300 can implement the functions of the first generation unit 301 and the second generation unit 302 by software (i.e., a central processing unit (CPU) executing a program), an electric circuit, or a plurality of electric circuits or a plurality of software. The processor 300 may implement the functions of the first generation unit 301 and the second generation unit 302 by distributed processing with an external device such as an external PC.

The processor 300 can implement the function of the storage unit 303 by a memory such as a hard disk drive (HDD) or a solid state drive (SSD). The processor 300 can implement the function of the output unit 304 by an interface or the like.

The first generation unit 301 generates gas spatial distribution information D based on the gas information G from the first detector 100. The gas spatial distribution refers to the distribution of gases in space. The gas spatial distribution information is information on the spatial distribution of gas in space.

The gas information G output from the first detector 100 includes information on gas around each of the plurality of inlets. The first generation unit 301 can generate the gas spatial distribution information D from the information on the gas around each of the plurality of inlets, included in the gas information G. In other words, the gas spatial distribution information D is data indicating which gas is sucked from which of the plurality of inlets as gas distribution.

The second generation unit 302 receives the object position information Im from the second detector 200. The second generation unit 302 receives the gas spatial distribution information D from the first generation unit 301. The second generation unit 302 retrieves the first relative position information N from the storage unit 303.

The second generation unit 302 generates the second relative position information Q indicating the gas spatial distribution relative to the position of an object based on the object position information Im, the spatial distribution information D of the gas, and the first relative position information N.

The second generation unit 302 can output the second relative position information Q to an external device using the output unit 304. Examples of the external device here include an external PC that performs a predetermined process using the second relative position information Q, a display apparatus that displays the second relative position information Q, a storage device that stores the second relative position information Q, a communication device that transmits and receives the second relative position information Q.

### Operation of Gas Detection Device according to First Embodiment

There are conventional gas detection devices without the capability of acquiring gas spatial distribution information relative to the position of an object. The usage of such a gas detection device may be limited because a target gas discharged from a detection target (e.g., an object) and a gas in the environment surrounding the detection target are mixed in the detection result and cannot be distinguished from each other.

By contrast, the gas detection device 500 according to the present embodiment can output the second relative position information Q indicating the gas spatial distribution relative to the position of an object. Accordingly, the gas detection device 500 can individually detect the gas at the detection target and the gas in the environment surrounding the detection target. For example, the gas detection device 500 can individually detect a first position where a first gas is generated and a second position where a second gas different from the first gas is generated in space. According to the present embodiment, the gas detection device 500 capable of outputting the gas spatial distribution information D is provided.

Since the gas detection device 500 can output the second relative position information Q indicating the gas spatial distribution relative to the position of an object, the uses thereof can be expanded. Examples of the expanded uses include the use in searching for an odor source and the use in visualizing an inflow path of air pollution in a clean room.

The gas detection device 500 can also detect a temporal change (dynamic change) in the gas spatial distribution relative to the position of the object using the gas information G output in time series from the first detector 100 and the object position information Im output in time series from the second detector 200. Since the gas detection device 500 can detect a temporal change in the gas spatial distribution relative to the position of the object, the use of the gas detection device 500 can be further expanded.

The object information acquisition device 220 of the second detector 200 may be a distance-measuring device that outputs information on the distance to the object. The object information acquisition device 220 may include both the image capturing device and the distance-measuring device. The distance-measuring device is, for example, a laser imaging detection and ranging (LIDAR) device or a stereo camera.

A LIDAR device emits laser light radiating in a pulsed manner and measures the distance to an object based on return light that is the emitted laser light being reflected or scattered by the object.

A stereo camera is a camera which captures images of an object from a plurality of directions in parallel so as to acquire information also on the depth direction.

The second detector 200 can output position information of an object in a three-dimensional space when a distance-measuring device is included therein. The gas detection device 500 can acquire the gas spatial distribution and three-dimensional relative position information of the gas relative to the object by using the position information of the object in the three-dimensional space, thereby further expanding its uses.

### Example of Object Information

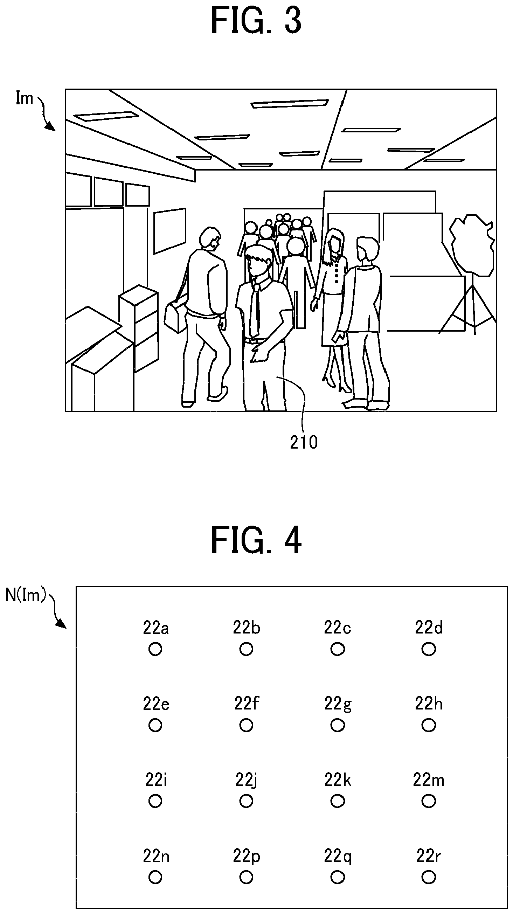

FIG. 3 is a diagram illustrating an example of the object position information Im. The object position information Im illustrated in FIG. 3 is an image captured by the image capturing device of the second detector 200 (refer to FIG. 1). The object position information Im includes a plurality of image regions 210 corresponding to a plurality of objects. The object position information Im may be a spherical image (omnidirectional image).

### Example of First Relative Position Information

FIG. 4 is a diagram illustrating an example of the first relative position information N. The first relative position information N is, for example, information indicating a position of each of the plurality of inlets in the captured image serving as the object position information Im output by the second detector 200. As illustrated in FIG. 4, the first relative position information N includes inlet positions 22a to 22r. The inlet positions 22a to 22r represent the positions of the inlets in the object position information Im.

In the present embodiment, the positions of the inlet positions 22a to 22r in the object position information Im are determined in advance. In other words, the first relative position information N is preliminarily generated and stored in the storage unit 303. In order to determine the first relative position information N in advance, in the present embodiment, first, the second detector 200 (see FIG. 1) performs capturing of an image including the inlets. Thereafter, coordinate information indicating the position of each of the inlets in the image captured by the second detector 200 is stored. In the present embodiment, this coordinate information is used as the first relative position information N.

The gas detection device 500 (see FIG. 1) refers to the first relative position information N and identifies the position of each of the inlets in the captured image serving as the object position information Im so as to associate the gas spatial distribution information D based on the gas information G with the captured image. As a result, the gas detection device 500 can generate the second relative position information Q indicating the gas spatial distribution relative to the position of an object.

In the present embodiment, since the first relative position information N is preliminarily generated and stored, the calculation load and the calculation time can be reduced as compared with a case where the first relative position information N is acquired by calculation every time the second detector 200 performs imaging.

### Example of Second Relative Position Information

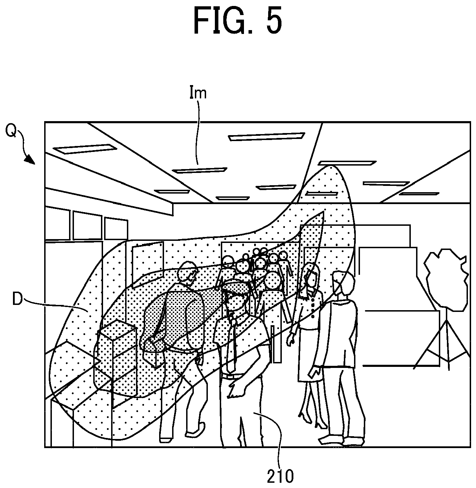

FIG. 5 is a diagram illustrating the second relative position information Q according to the first embodiment. As illustrated in FIG. 5, the gas spatial distribution information D is superimposed on the captured image serving as the object position information Im. In the gas spatial distribution information D in FIG. 5, differences in shading pattern indicate differences in the type of gas in the space, the component content in the gas, and the like. For example, in the gas spatial distribution information D, an area in which a first gas is detected is indicated by a dense dot pattern.

Further, in the gas spatial distribution information D, an area in which a second gas different from the first gas is detected is indicated by a sparse dot pattern.

### Configuration of First Detector

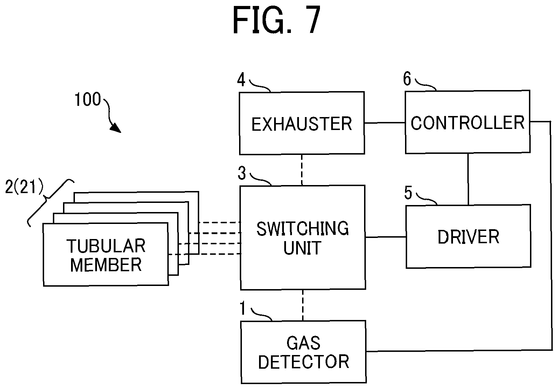

A description is given of an example of the overall configuration of the first detector 100 with reference to FIGS. 6 and 7.

FIG. 6 is a schematic diagram illustrating an overall configuration of the first detector 100. FIG. 7 is a block diagram illustrating the overall configuration of the first detector 100.

As illustrated in FIGS. 6 and 7, the first detector 100 includes a gas detector 1, a plurality of tubular members 2a to 2h (collectively "tubular members 2"), a switching unit 3, an exhauster 4, a driver 5, and a controller 6. In FIG. 7, among the lines connecting the blocks representing these components, the broken connection lines indicate that the components are connected such that gas can flow. On the other hand, the solid connection lines indicate electrical connection.

The first detector 100 takes in the gas present around respective inlets 21a to 21h (collectively "inlets 21") of the plurality of tubular members 2 through the individual inlets 21 as the gas detector 1 and the exhauster 4 perform suction. In the first detector 100, the gas detector 1 sequentially and individually detects a plurality of gases flowing through the plurality of tubular members 2 while the switching unit 3 switches the path leading to the detection position of the gas detector 1. In this specification, detection of a gas refers to detecting the kind and the amount of a small number of molecules in the gas, or detecting the odor of the gas.

The first detector 100 detects the gas present around each of the plurality of inlets 21, thereby detecting the gas spatial distribution in indoor space 600 such as a room. However, the space in which the gas spatial distribution can be detected by the first detector 100 is not limited to indoor spaces and may be an outdoor space. The gas spatial distribution refers to distribution of gases in space. The first detector 100 can output such a detection result as the gas information G. Note that the indoor space 600 is not a component of the first detector 100.

The gas detector 1 detects the gas flowing thereto. The gas detector 1 is, for example, an ion mobility spectrometer that detects a gas by ion mobility spectrometry (IMS). Ion mobility spectrometry is a method for measuring ion mobility by utilizing the fact that the mobility of an ionized molecule in a high electric field varies depending on the molecule.

The gas detector 1 ionizes a gas to be detected among a plurality of gases flowing through the plurality of tubular members 2 by, for example, ultraviolet ray irradiation, corona discharge, or radioisotope radiation. The gas detector 1 filters and detects ions in the ionized gas. The gas detector 1 has a suction function and can supply, by suction, a gas to be detected among a plurality of gases flowing through the plurality of tubular members 2 to the position of the gas detector 1.

The gas detector 1 may include a field asymmetric ion mobility spectrometer, an ion mobility spectrometer, or the like. There is a property that the mobility of the ions is not proportional to the electric field in a strong electric field. Using such a property, a field asymmetric ion mobility spectrometer causes ions to pass through an electric field having a high-voltage asymmetric waveform, thereby separating and detecting the ions. An ion mobility spectrometer performs detection based on the difference in moving speed of ions caused to fly in a gas applied with an electric field.

The gas detector 1 includes a tube 10 and a filter 11. The tube 10 serves as a flow path that guides the gas exhausted from the switching unit 3 to the gas detector 1. The filter 11 is provided at a stage preceding a processing unit for ionizing a gas to be detected, and diffuses the gas flowing through the tube 10. The gas detector 1 detects the gas having passed through the filter 11.

Even when the detection accuracy varies depending on the direction in which the gas flows to the gas detector 1, since the gas detector 1 detects the gas diffused through the filter 11, the first detector 100 can reduce variations in the detection result due to the variations in the direction in which the gas flows. As a result, the first detector 100 can secure a high gas detection accuracy.

The gas detector 1 outputs the gas detection result to the controller 6.

Each of the plurality of tubular members 2 serves as a flow path for the gas sucked in through the corresponding inlet 21. Each of the plurality of tubular members 2 has the inlet 21 at one end. The other end of each of the plurality of tubular members 2 is connected to the switching unit 3 such that gas can flow therethrough.

The plurality of tubular members 2 includes the tubular member 2a, the tubular member 2b, the tubular member 2c, the tubular member 2d, the tubular member 2e, the tubular member 2f, the tubular member 2g, and the tubular member 2h.

The tubular member 2a has the inlet 21a. The tubular member 2b has the inlet 21b. The tubular member 2c has the inlet 21c. The tubular member 2d has the inlet 21d. The tubular member 2e has the inlet 21e. The tubular member 2f has the inlet 21f. The tubular member 2g has the inlet 21g. The tubular member 2h has the inlet 21h.

Each of the plurality of tubular members 2 is, for example, a flexible and bendable resin hose. The plurality of tubular members 2 is laid along a wall or a ceiling of the indoor space 600 so that the respective inlets 21 are at different positions. The gas around each of the plurality of inlets 21 is sucked from the inlet 21 and flows through the tubular member 2 toward the switching unit 3.

The number of the plurality of tubular members 2 is not limited to eight (the tubular member 2a to the tubular member 2h) but can be appropriately changed in accordance with the spatial resolution of gas detection, the size of the indoor space 600 in which gas is detected, and the like.

The plurality of tubular members 2 is not limited to hoses and may be tubes, ducts, or the like. The material of the plurality of tubular members 2 is not limited to resin and may be, for example, metal.

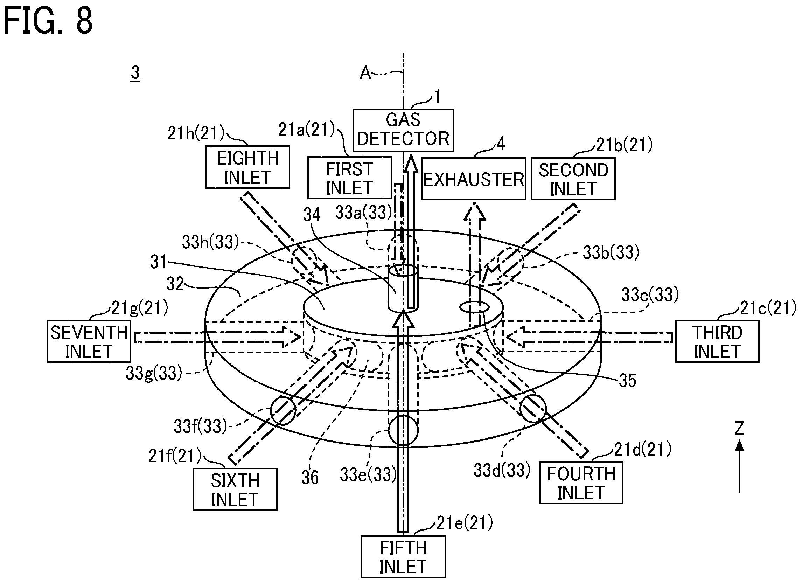

The switching unit 3 can switch the gas to be detected by the gas detector 1 among the gases flowing through the plurality of tubular members 2. The configuration of the switching unit 3 will be described in detail later with reference to FIGS. 8 to 11.

The exhauster 4 exhausts the gas flowing through the switching unit 3. The exhauster 4 includes, for example, a vacuum pump and can exhaust the gas flowing thereto to the outside of the switching unit 3 by suction by the vacuum pump. The vacuum pump used in the present embodiment can be any of various vacuum pumps such as a rotary pump, a diffusion pump, and a swing piston pump. The exhauster 4 may perform exhaust using a component other than a pump, such as an ejector mechanism.

The exhauster 4 is coupled, via the switching unit 3, to each of the tubular members 2 other than the detection targets tubular member 2 such that gas can flow. The detection target tubular member 2 refers to the tubular member 2 in which the gas to be detected by the gas detector 1 flows. The exhauster 4 sucks, toward the switching unit 3, the gases flowing through the plurality of tubular members 2 other than the gas to be detected by the gas detector 1 and then exhausts the gas to the outside of the switching unit 3.

The driver 5 drives the switching unit 3. For example, the driver 5 is a motor that rotates a rotator 31 (see FIG. 8) included in the switching unit 3. A rotation shaft of the motor is coupled to the rotator 31 (see FIG. 8) included in the switching unit 3 via, for example, a coupling so that the rotator 31 can rotate on the rotation shaft.

The motor serving as the driver 5 may be, for example, a stepping motor, an alternating current (AC) motor, or a direct current (DC) motor. From the viewpoint of facilitating intermittent rotation control of the rotator 31 (see FIG. 8) included in the switching unit 3, the use of a stepping motor is preferable. The intermittent rotation of a rotator means that the rotator alternately rotates and stops the rotation.

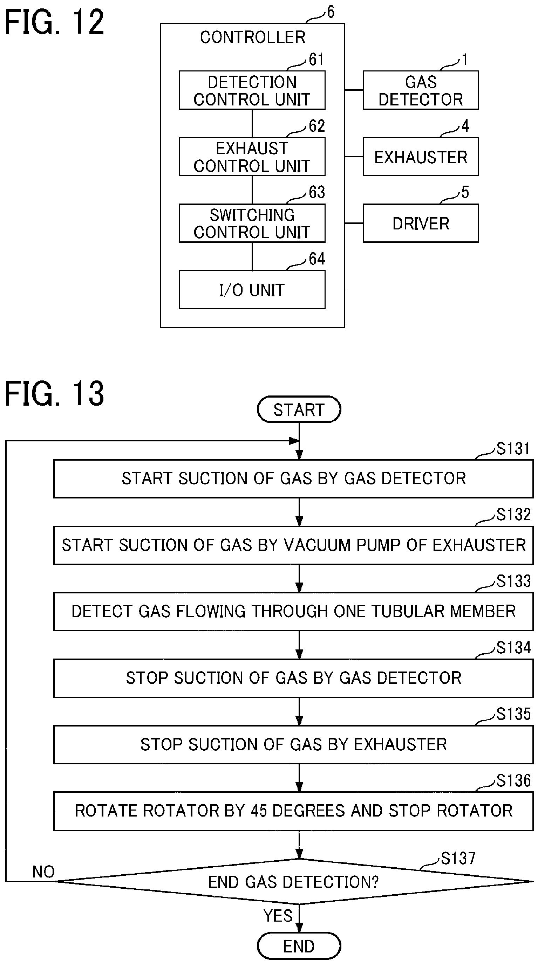

The controller 6 controls the operation of each of the gas detector 1, the exhauster 4, and the driver 5. The controller 6 will be described in detail with reference to FIG. 12.

As described above, the first detector 100 includes the plurality of tubular members 2 having the inlets 21 disposed at different positions in a space and switches the gas to be detected by the gas detector 1 among the plurality of gases flowing through the plurality of tubular members 2. Accordingly, the first detector 100 can sequentially and individually detect the gas around each of the plurality of inlets 21.

For example, a gas detection device that detects gas at only one location in a space is not able to detect the gas spatial distribution. For detecting gas spatial distribution, it is also conceivable to use a movable gas detection device (or a movable detection unit included in a gas detection device) that detects the gas at a plurality of positions in a space while moving around in the space. However, in this method, the time at which the gas is detected differs among the plurality of positions, and the gas spatial distribution is not detected in real time in some cases. In addition, due to the movement of the gas detection device or the detection unit included in the gas detection device, the air present in the space is stirred, and the accuracy in detection of the gas spatial distribution is not high in some cases.

The first detector 100 according to the present embodiment can acquire information on the gas spatial distribution using the individual detection results obtained for each of the plurality of inlets 21 disposed at different positions. Accordingly, the present embodiment can provide the gas detection device capable of detecting the gas spatial distribution.

In the present embodiment, at the time of detection by the gas detector 1, among the gases flowing through the plurality of tubular members 2, the gas other than the gas to be detected by the gas detector 1 is also sucked. Accordingly, the first detector 100 according to the present embodiment can minimize the time difference in detection of the gases flowing through the plurality of tubular members 2, that is, detect the gases flowing through the plurality of tubular members 2 at substantially the same timing. As a result, in the present embodiment, high accuracy can be secured in the detection of the gas spatial distribution.

By using the first detector 100, the gas present at a plurality of positions in a space can be detected under the same or similar condition, and thus the gas spatial distribution can be analyzed with high accuracy, high efficiency, or easily.

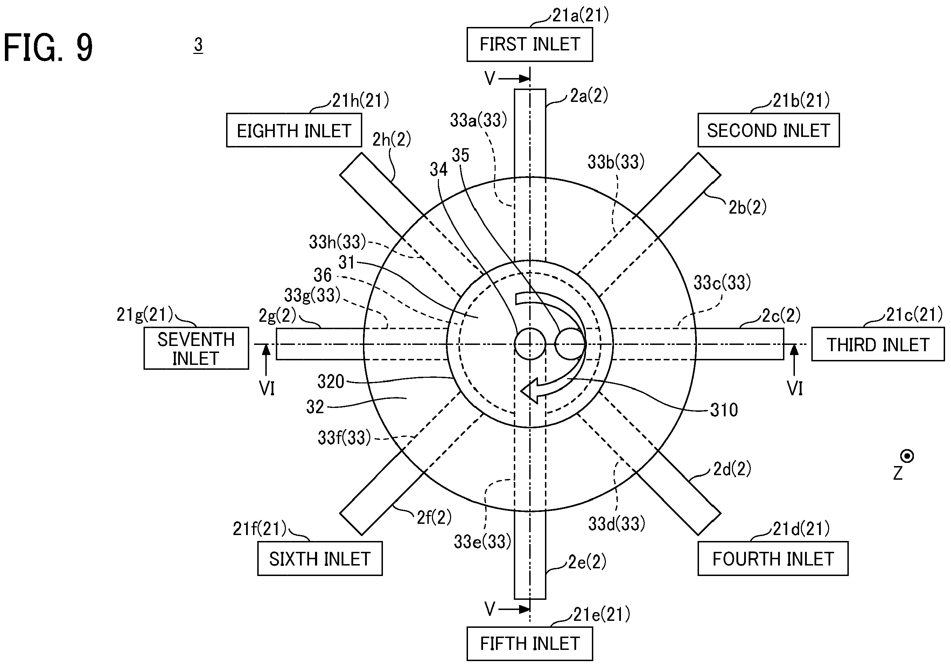

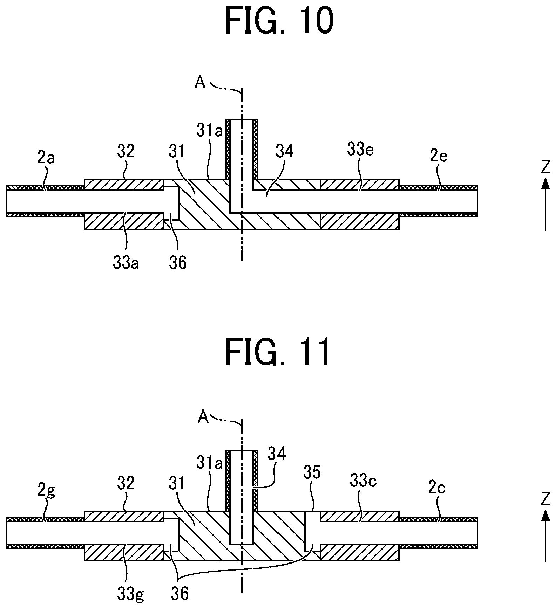

A description is given below of the configuration of the switching unit 3, with reference to FIGS. 8 to 11. FIG. 8 is a perspective view illustrating an example of the configuration of the switching unit 3. FIG. 9 is a top view illustrating the example of the configuration of the switching unit 3. FIG. 10 is a cross-sectional view taken along the line V-V in FIG. 9. FIG. 11 is a cross-sectional view taken along the line VI-VI in FIG. 9.

As illustrated in FIGS. 8 to 11, the switching unit 3 includes the rotator 31 and a support body 32.

The rotator 31 includes a first flow path 34, a second flow path 35, and a groove 36. The support body 32 includes a plurality of third flow paths 33a to 33h (collectively referred to as "third flow paths 33") and a through hole 320 (see FIG. 9). For example, each of the first flow path 34, the second flow path 35, and the third flow paths 33 is a duct. FIGS. 8 to 11 illustrate a state in which the rotator 31 is inserted into the through hole 320 of the support body 32.

Each of the rotator 31 and the support body 32 can include, for example, metal. However, the material of each of the rotator 31 and the support body 32 is not particularly limited, and, for example, resin may be used.

The plurality of third flow paths 33a to 33h is individually coupled to the plurality of tubular members 2a to 2h such that gas can flow. Each of the plurality of tubular members 2 has the inlet 21. In the present embodiment, the switching unit 3 switches the tubular member 2 that communicates with the first flow path 34 via the third flow path 33 among the plurality of tubular members 2 using the rotation of the rotator 31. Thus, the switching unit 3 can switch the gas to be detected by the gas detector 1 among the gases flowing through the plurality of tubular members 2.

As illustrated in FIGS. 8 and 9, the rotator 31 has a columnar shape and is rotatable about a columnar axis A in a rotation direction 310 (clockwise) in FIG. 9. The columnar axis A corresponds to the rotation axis of the rotator 31.

Note that the rotation direction 310 may be counterclockwise.

The support body 32 has a columnar shape and includes the through hole 320 at the center in a top view. The support body 32 supports the rotator 31 inserted into the through hole 320 to rotate. In this specification, the top view refers to viewing the rotator 31 or the support body 32 in a direction along the columnar axis A that is common to the rotator 31 and the support body 32.

The first flow path 34 is a flow path that guides, to the gas detector 1, a gas to be detected by the gas detector 1 among the plurality of gases flowing from the plurality of tubular members 2 to the plurality of third flow paths 33.

As illustrated in FIGS. 10 and 11, the first flow path 34 extends along the direction of the columnar axis A of the rotator 31 and also extends from the columnar axis A of the rotator 31 toward the outer periphery of the rotator 31 (in the diameter direction) such that the upper side of the rotator 31 communicates with the outer periphery thereof. In this specification, the upper side corresponds to the +Z direction along the Z axis indicated in FIGS. 8 to 11. The +Z direction refers to the direction to which the arrow orients in the Z direction.

In the present embodiment, a part of the first flow path 34 protrudes upward from a top face 31a (see FIG. 10) of the rotator 31 at the center of the rotator 31 in the top view. The gas detector 1 is disposed to detect the gas discharged to the upper side of the rotator 31 through the first flow path 34. In the present embodiment, the gas detector 1 is provided so as to detect the gas that has been exhausted above the rotator 31, passed through the tube 10, and passed through the filter 11.

The second flow path 35 is a flow path for discharging the gases other than the gas detected by the gas detector 1 among the plurality of gases flowing from the plurality of tubular members 2 to the plurality of third flow paths 33. As illustrated in FIG. 11, the second flow path 35 is a flow path that connects a portion above the top face 31a of the rotator 31 and the groove 36.

The groove 36 is formed in the outer periphery of the rotator 31 along the circumferential direction (in the shape of column). In the present embodiment, the groove 36 is formed in the outer periphery of the rotator 31, except a region where the first flow path 34 is formed, along the circumferential direction of the rotator 31. In a state where the rotator 31 is inserted into the through hole 320 of the support body 32, the gases flowing from the plurality of tubular members 2 can flow in the circumferential direction in a flow path space which is a space formed by the groove 36 and an inner side face of the through hole 320.

The exhauster 4 exhausts the gas through the second flow path 35. In the present embodiment, the vacuum pump included in the exhauster 4 is coupled to one end of the second flow path 35 located on the top face 31a side of the rotator 31. Owing to the suction of the vacuum pump, the gas flowing through the above-mentioned flow path space is discharged to the outside of the rotator 31 through the second flow path 35.

The plurality of third flow paths 33 in the support body 32 include the third flow path 33a, the third flow path 33b, the third flow path 33c, the third flow path 33d, the third flow path 33e, the third flow path 33f, the third flow path 33g, and the third flow path 33h. Each of the plurality of third flow paths 33 communicates with either the first flow path 34 or the second flow path 35 in the rotator 31, which is inserted into the through hole 320. The path with which each of the third flow paths 33 communicates is switched by the rotation of the rotator 31.

In the present embodiment, the plurality of third flow paths 33 is disposed radially around the columnar axis A in the top view (see FIG. 9). In the top view, the angles formed by the central axes of the third flow paths 33 are substantially equal. Since there are eight third flow paths 33, the angle formed by the central axes of the adjacent two of third flow paths 33 is 45 degrees. The rotator 31 is intermittently rotated by 45 degrees by the driver 5.

In the present embodiment, the plurality of third flow paths 33 has an equal length. The wording "equal length" means "substantially equal length." The wording "substantially equal length" or "lengths are substantially equal" does not require that the lengths be strictly equal to each other, but means that a difference to be generally recognized as a tolerance is allowed. The difference in length to be generally recognized as a tolerance is, for example, one tenth or less of a target length (here, the length of the third flow path 33). This also applies to the case where the term "substantially equal length" is used in the description below.

In the present embodiment, making the lengths of the plurality of third flow paths 33 substantially equal to each other is advantageous in minimizing the influence of the length of the third flow path 33 on the gas detection accuracy of the gas detector 1. Accordingly, in the present embodiment, a high accuracy can be secured in the detection of the gas spatial distribution by the first detector 100.

Further, making the lengths of the plurality of tubular members 2 substantially equal to each other is advantageous in that the gases individually flowing through the tubular members 2 and the third flow paths 33 is always replaced at the same time. Thus, in the present embodiment, the gases distributed in the space can be detected at the same time. In other words, in the present embodiment, the gas spatial distribution can be detected in real time. However, the present embodiment is not limited to a configuration in which the third flow paths 33 have an equal length. Further, the present embodiment is not limited to a configuration in which the plurality of tubular members 2 has an equal length.

The number of the plurality of third flow paths 33 can be changed in accordance with the number of the plurality of tubular members 2. The angles formed by the central axes of the plurality of third flow paths 33 in the top view is not necessarily substantially equal to each other and may be freely-set angles different from each other.

The third flow path 33 that communicates with the first flow path 34 can be switched among the plurality of third flow paths 33 by the rotation of the rotator 31. Similarly, the third flow path 33 that communicates with the second flow path 35 via the groove 36 can be switched among the plurality of third flow paths 33 by the rotation of the rotator 31.

In the state illustrated in FIGS. 8 to 11, as a result of the rotation of the rotator 31, the third flow path 33e communicates with the first flow path 34. As illustrated in FIG. 10, the gas sucked into the tubular member 2e is guided from the tubular member 2e through the third flow path 33e to the first flow path 34, exhausted from the first flow path 34 to an area above the rotator 31, and then detected by the gas detector 1.

By contrast, each of the third flow paths 33a, 33b, 33c, 33d, 33f, 33g, and 33h communicates with the space formed by the groove 36 and the inner side face of the through hole 320.

As illustrated in FIG. 11, the gas sucked into the tubular member 2c is guided from the tubular member 2c to the second flow path 35 through the third flow path 33c and the groove 36, and is discharged from the second flow path 35 to the area above the rotator 31.

The gas flowing through each of the tubular members 2a, 2b, 2d, 2f, 2g, and 2h is guided to the second flow path 35 through the groove 36 and is discharged from the second flow path 35 to the area above the rotator 31, similar to the gas flowing through the tubular member 2c.

As described above, the gas flowing through each of the tubular members 2a, 2b, 2c, 2d, 2f, 2g, and 2h is not detected by the gas detector 1.

To switch the state illustrated in FIGS. 8 to 11 to the state in which the gas flowing through the tubular member 2f is to be detected, the switching unit 3 intermittently rotates the rotator 31 by 45 degrees in the rotation direction 310. As a result, the first flow path 34 communicates with the third flow path 33f. Therefore, the gas sucked into the tubular member 2f through the inlet 21f is guided from the tubular member 2f to the first flow path 34 through the third flow path 33f, discharged from the first flow path 34 to the area above the rotator 31, and then detected by the gas detector 1.

As described above, the switching unit 3 can switch the gas to be detected by the gas detector 1 among the gases flowing through the plurality of tubular members 2 by rotating the rotator 31 and switching the third flow path 33 that communicates with the first flow path 34.

In the present embodiment, the gases flowing through the plurality of tubular members 2 are collected into the switching unit 3 and the rotator 31 of the switching unit 3 is rotated, so as to switch the gas to be detected by the gas detector 1 among the gases flowing through the plurality of tubular members 2. Thus, in the present embodiment, the gas detected by the gas detector 1 can be switched with a simple configuration, and the first detector 100 can be compact.

A description is given of an example of the functional configuration of the controller 6.

FIG. 12 is a block diagram illustrating an example of the functional configuration of the controller 6. The controller 6 includes a detection control unit 61, an exhaust control unit 62, a switching control unit 63, and an input and output (I/O) unit 64.

The functional units of the controller 6 may be implemented by an electric circuit, or some or all of the functional units may be implemented by software (i.e., a CPU executing a program). Further, the functional units of the controller 6 may be implemented by a plurality of circuits, a plurality of pieces of software, or distributed processing with an external device such as a PC.

The detection control unit 61 controls the operation of the gas detector 1. For example, the detection control unit 61 can control start and stop of suction by the gas detector 1, start and stop of acquisition of detection data, output of a detection result to an external device via the I/O unit 64, and the like. The external device here is a PC, a display, a memory, a network device, or the like.

The exhaust control unit 62 controls the operation of the exhauster 4. For example, the exhaust control unit 62 can control the start or stop of suction by the vacuum pump of the exhauster 4, the flow rate of the gas sucked by the vacuum pump, and the like.

The switching control unit 63 controls the operation of the driver 5. For example, the switching control unit 63 can control the start of rotation of the rotator 31 by the driver 5, the rotation angle of the rotator 31, the rotation speed of the rotator 31, and the like.

The I/O unit 64 controls input and output of signals or data between the controller 6 and an external device. The external device here is, for example, the gas detector 1, the exhauster 4, or the driver 5.

In the present embodiment, the switching control unit 63 rotates the driver 5 by 45 degrees, thereby rotating the rotator 31 about the columnar axis A by 45 degrees. Accordingly, the switching control unit 63 can sequentially switch the third flow path 33 that communicates with the first flow path 34 among the plurality of third flow paths 33, so as to sequentially switch the gas to be detected by the gas detector 1 among the gases flowing through the plurality of tubular members 2.

In the present embodiment, the switching control unit 63 stops the operation of the driver 5 during a period in which the gas detector 1 detects the gas. In other words, the switching control unit 63 intermittently drives the driver 5, and the gas detector 1 performs gas detection during a period in with which the driver 5 stops the operation. With this operation, in the present embodiment, the state of the gas detected by the gas detector 1 is kept unchanged during the period in which the gas detector 1 detects the gas. Accordingly, a high accuracy can be secured in the detection by the gas detector 1. However, the switching control unit 63 may not necessarily stop the operation of the driver 5 during the period in which the gas detector 1 detects the gas, and may continuously drive the driver 5.

In the present embodiment, during the period in which the driver 5 is driven by the switching control unit 63, the detection control unit 61 stops the suction by the gas detector 1, and the exhaust control unit 62 stops the suction by the vacuum pump. Such a control operation can avoid mixing of gases while the rotator 31 rotates, and secure a high accuracy in the detection by the first detector 100. However, the detection control unit 61 does not necessarily stop the suction by the gas detector 1 during the period in which the driver 5 is driven by the switching control unit 63, and the gas detector 1 may constantly perform the suction. In addition, the exhaust control unit 62 does not necessarily stop the suction by the vacuum pump during the period in which the driver 5 is driven by the switching control unit 63, and the vacuum pump may constantly perform the suction.

A description is given of an example of operation of the first detector 100.

FIG. 13 is a flowchart of a process executed by the first detector 100. For example, the first detector 100 starts the process illustrated in FIG. 13 in response to an input of instruction operation to start the detection by the first detector 100, using an operation device of the first detector 100.

First, in step S131, the first detector 100 starts suction of gas by the gas detector 1.

Subsequently, in step S132, the first detector 100 starts suction of gas by the vacuum pump of the exhauster 4.

Subsequently, in step S133, the first detector 100 detects, with the gas detector 1, the gas flowing from one tubular member 2 communicating with the first flow path 34, among the plurality of tubular members 2. The first detector 100 outputs the gas information G of the detected gas.

Subsequently, in step S134, the first detector 100 stops the suction of gas by the gas detector 1 after the detection operation by the gas detector 1 ends.

Subsequently, in step S135, the first detector 100 stops the suction of gas by the vacuum pump of the exhauster 4.

Subsequently, in step S136, the first detector 100 drives, with the driver 5, the switching unit 3 to rotate the rotator 31 by 45 degrees in the rotation direction 310 (refer to FIG. 9). After the rotator 31 rotates 45 degrees in the rotation direction 310, the first detector 100 stops the rotation of the rotator 31.

Subsequently, in step S137, the first detector 100 determines, with the controller 6, whether to end the gas detection. For example, the controller 6 can determine whether to end the detection of the gas in response to an input of operation to end the detection by the first detector 100, using the operation device of the first detector 100. However, the controller 6 may determine whether to end the detection of the gas based on a predetermined condition. The predetermined condition is, for example, whether or not the time length of the detection exceeds a predetermined time length or whether or not the number of times the detection has been performed reaches a predetermined number.

When it is determined in step S137 that the gas detection operation is to be ended (step S137, YES), the first detector 100 ends the gas detection operation. On the other hand, when it is determined in step S137 that the operation is not to be ended (NO in step S137), the first detector 100 again performs the process from step S131.

As described above, the first detector 100 can output the gas information G on the gas sucked from each of the respective inlets 21 of the plurality of tubular members 2.

### Second Embodiment

A description is given of a gas detection device 500a according to a second embodiment. Note that the components equivalent to those described in the first embodiment are given the same or similar reference numerals, and redundant descriptions are omitted as appropriate. This also applies to other embodiments described below.

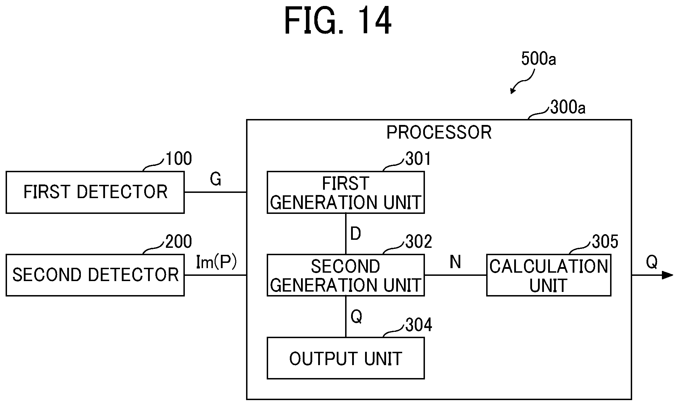

FIG. 14 is a block diagram illustrating an example of the functional configuration of a processor 300a included in the gas detection device 500a. Other than the processor 300a, components of the gas detection device 500a are the same as those of the gas detection device 500 according to the first embodiment. As illustrated in FIG. 14, the processor 300a includes a calculation unit 305.

The processor 300a can implement the function of the calculation unit 305 by software (a CPU executing a program), an electric circuit, a plurality of electric circuits, or a plurality of pieces of software. The processor 300a may implement the function of the calculation unit 305 by distributed processing with an external device.

The calculation unit 305 receives inlet position information P indicating the position of each of the plurality of inlets from the second detector 200. For example, the image capturing device of the second detector 200 performs image capturing such that an image of each of the plurality of inlets is included in the captured image used as the object position information Im. The inlet position information P is image region information corresponding to each of the plurality of inlets included in the captured image used as the object position information Im.

The calculation unit 305 calculates the first relative position information N based on the inlet position information P.

For example, the gas detection device 500 performs, with the second detector 200, image processing on the captured image, to detect the position of each of the plurality of inlets in the captured image. Based on this detection result, the gas detection device 500 can obtain the first relative position information N.

When the first relative position information N is determined in advance as in the first embodiment, the position and the image capturing direction of the second detector 200 need to be unchanged. If the position and the image capturing direction of the second detector 200 are changed, the gas detection device 500 needs to determine the first relative position information N again.

In the present embodiment, the first relative position information N is acquired by calculation based on the captured image every time the second detector 200 performs image capturing. Accordingly, it is not necessary to determine the first relative position information N again even if the position and the image capturing direction of the second detector 200 change. Therefore, in the present embodiment, the gas can be detected while freely changing the position and the image capturing direction of the second detector 200, and the degree of freedom of detection by the gas detection device 500 can be improved. Further, the present embodiment provides effects similar to those provided by the first embodiment described above.

### Third Embodiment

### Configuration Example of Gas Detection System 700

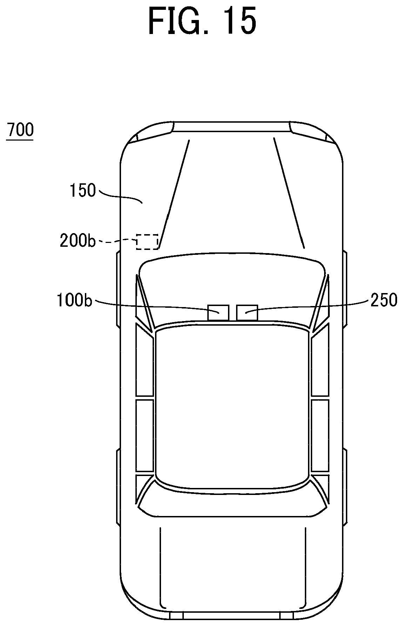

FIG. 15 is a diagram illustrating a configuration of a gas detection system 700 according to a third embodiment. The gas detection system 700 includes a traveling body 150, a position information acquisition unit 200b, and an image capturing device 250.

The traveling body 150 includes a first detector 100b that outputs gas information Gb on an inhaled gas and is movable. The first detector 100b includes, for example, the above-described field asymmetric ion mobility spectrometer or the ion mobility spectrometer. The term "traveling body" refers to an object capable of moving. In this embodiment, the traveling body 150 is an automobile. However, the traveling body 150 is not limited to an automobile, and may be a flying body such as a drone, an automated guided vehicle, a ship, or a train.

The position information acquisition unit 200b outputs position information S indicating the position of the first detector 100b moved by the traveling body 150. In the present embodiment, the position information acquisition unit 200b includes a global position system receiver (hereinafter "GPS receiver"). The GPS receiver acquires its own position information based on a signal received from a global position system. The position information acquisition unit 200b can output position information S indicating the position of the first detector 100b acquired by the GPS receiver.

The image capturing device 250 can output a captured image T of the surroundings of the traveling body 150. The image capturing device 250 is a component different from the image capturing device included in the second detector 200 in the above-described first embodiment.

The image capturing device 250 is a camera including an imaging element, such as a CCD or a CMOS, and a lens that forms an image of a subject on an imaging surface of the imaging element. The image capturing device 250 can perform image capturing in synchronization with the acquisition of the position information S by the position information acquisition unit 200b. The image capturing device 250 outputs the captured image T in association with the position information S output from the position information acquisition unit 200b.

### Example of Functional Configuration of Processor 300b

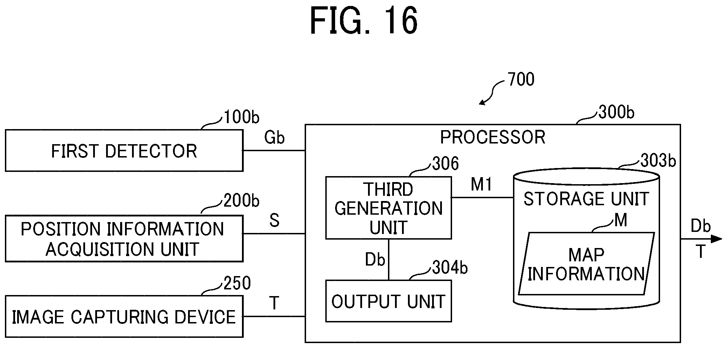

FIG. 16 is a block diagram illustrating an example of the functional configuration of a processor 300b included in the gas detection system 700. The processor 300b includes a third generation unit 306, a storage unit 303b, and an output unit 304b.

The processor 300b can implement the function of the third generation unit 306 by software (a CPU executing a program), an electric circuit, a plurality of electric circuits, or a plurality of pieces of software. Alternatively, the processor 300b may implement the function of the third generation unit 306 by distributed processing with an external device. The processor 300b can implement the function of the storage unit 303b by a memory such as an HDD or an SSD. The processor 300b can implement the function of the output unit 304b by an interface or the like.

The third generation unit 306 generates gas spatial distribution information Db based on the gas information Gb from the first detector 100b and the position information S from the position information acquisition unit 200b.

The storage unit 303b stores map information M. The third generation unit 306 refers to the map information M stored in the storage unit 303b and retrieves map information M1 of a specified area therefrom. For example, the third generation unit 306 retrieves, from the map information M, map information on an area including the position of the first detector 100b indicated by the position information S on the first detector 100b, received from the position information acquisition unit 200b, as the map information M1. The third generation unit 306 can generate the gas spatial distribution information Db associated with the map information M1. The specified area is not limited to a part of the area corresponding to the map information M, but may be the entire area corresponding to the map information M.

The output unit 304b outputs, to an external device, the gas spatial distribution information Db received from the third generation unit 306 and the captured image T received from the image capturing device 250 associated with the gas spatial distribution information Db.

### Example of Gas Spatial Distribution Information

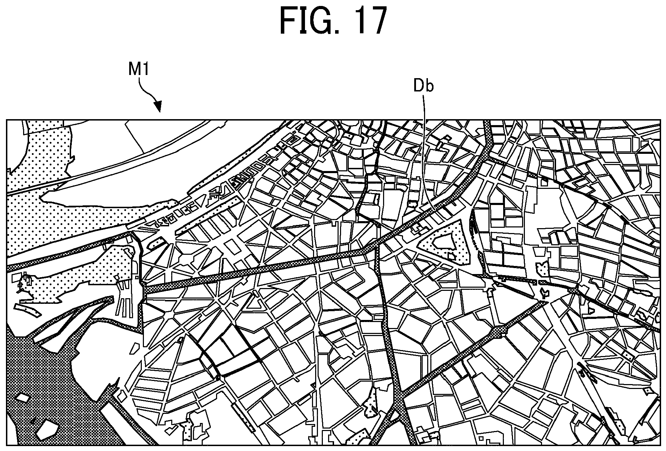

FIG. 17 is a diagram illustrating an example of the gas spatial distribution information Db associated with the map information M1. The map information M1 illustrated in FIG. 17 is, for example, information displayed on a screen of a display of a car-navigation system.

The map information M1 includes a road on which the traveling body 150 has traveled. In the map information M1, a dot shading pattern indicating the gas spatial distribution information Db is displayed on the road on which the traveling body 150 has traveled.

The gas spatial distribution information Db is acquired based on the gas information Gb output from the first detector 100b while the traveling body 150 travels. The difference in the type of dot shading patterns indicates the difference in the type of gas, the component content in the gas, and the like. For example, in the map information M1, an area in which a first gas is detected is indicated by a dense dot shading pattern. On the other hand, in the map information M1, an area in which a second gas different from the first gas is detected is indicated by a sparse dot shading pattern.

### Example of Captured Image

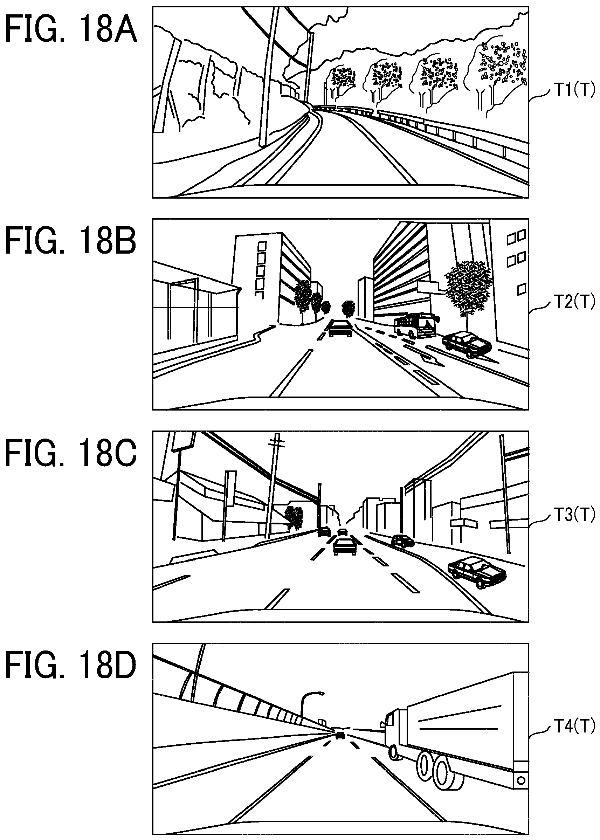

FIGS. 18A to 18D are diagrams illustrating examples of the captured image T captured by the image capturing device 250. FIG. 18A illustrates a captured image T1 captured when the traveling body 150 travels on a mountain road. FIG. 18B illustrates a captured image T2 when the traveling body 150 travels an urban area. FIG. 18C illustrates a captured image T3 when the traveling body 150 travels an urban area. FIG. 18D illustrates a captured image T4 when the traveling body 150 travels on an expressway.

The image capturing device 250 can acquire the captured image T of the scenery around the traveling body 150 in association with the position information S output from the position information acquisition unit 200b while the traveling body 150 travels. The gas detection system 700 can output the gas spatial distribution information Db in association with the surrounding scenery for each area in the map information M1. The gas detection system 700 may store the captured image T in the storage unit 303b or the like in association with the gas spatial distribution information Db.

As described above, the present embodiment provides the gas detection system 700 capable of outputting the gas spatial distribution information Db based on the gas information Gb from the first detector 100b and the position information S from the position information acquisition unit 200b.

In the present embodiment, the processor 300b outputs the gas spatial distribution information Db associated with the map information M1. Thus, the gas distribution in each area can be displayed in an easy-to-understand manner.

In the present embodiment, the position information acquisition unit 200b outputs the position information S based on the information received from the global position system. Thus, the position information S can be easily acquired. However, the method of acquiring the position information S by the position information acquisition unit 200b is not limited to the method using the global position system. Alternatively, for example, the position information acquisition unit 200b may cause a detector provided in the traveling body 150 to read a scale on a traveling path of the traveling body 150, to acquire the position information S.

In the present embodiment, the processor 300 outputs the captured image T associated with the position of the first detector 100b. As a result, the gas detection system 700 can provide information indicating the situation around the area where the gas is generated in an easy-to-understand manner.

Although some embodiments are described above, the above-described embodiments are merely examples and do not limit the present disclosure.

All of the numbers such as ordinal numbers and numerical values that indicate quantity in the above-described embodiments are examples used to describe the technologies to implement the embodiments of the present disclosure, and such numbers in the above-described embodiments are not intended to not limit the invention. In addition, the manners of coupling or connection between the elements described above are examples used to describe the technologies to implement the embodiments of the present disclosure, and how the elements are related to each other, coupled to each other, or connected to each other to implement the functionality in the present disclosure is not limited thereby.

Any one of the above-described operations may be performed in various other ways, for example, in an order different from the one described above.

The present invention can be implemented in any convenient form, for example using dedicated hardware, or a mixture of dedicated hardware and software. The present invention may be implemented as computer software implemented by one or more networked processing apparatuses. The processing apparatuses include any suitably programmed apparatuses such as a general purpose computer, a personal digital assistant, a Wireless Application Protocol (WAP) or third-generation (3G)-compliant mobile telephone, and so on. Since the present invention can be implemented as software, each and every aspect of the present invention thus encompasses computer software implementable on a programmable device. The computer software can be provided to the programmable device using any conventional carrier medium (carrier means). The carrier medium includes a transient carrier medium such as an electrical, optical, microwave, acoustic or radio frequency signal carrying the computer code. An example of such a transient medium is a Transmission Control Protocol/Internet Protocol (TCP/IP) signal carrying computer code over an IP network, such as the Internet. The carrier medium may also include a storage medium for storing processor readable code such as a floppy disk, a hard disk, a compact disc read-only memory (CD-ROM), a magnetic tape device, or a solid state memory device.

Aspects of the present disclosure are, for example, as follows.

According to Aspect 1, a gas detection device includes a first detector, a second detector, and a processor. The first detector outputs gas information being information on a gas sucked from each of a plurality of inlets arranged at different positions. The second detector outputs object position information indicating a position of an object. The processor outputs second relative position information indicating gas spatial distribution relative to the position of the object. The processor outputs the second relative position information based on gas spatial distribution information based on the gas information from the first detector, the object position information from the second detector, and first relative position information indicating a position of each of the plurality of inlets relative to the object.

According to Aspect 2, in the gas detection device of Aspect 1, the first relative position information is determined in advance.

According to Aspect 3, in the gas detection device of Aspect 1, the second detector is capable of outputting inlet position information indicating the position of each of the plurality of inlets, and the first relative position information is acquired based on the inlet position information.

According to Aspect 4, in the gas detection device of any one of Aspects 1 to 3, the second detector includes at least one of an image capturing device capable of outputting a captured image of the object and a distance-measuring device capable of outputting distance information relative to the object.

According to Aspect 5, a gas detection system includes a traveling body, a position information acquisition unit, and a processor. The traveling body includes a first detector to output gas information being information on inhaled gas. The position information acquisition unit outputs position information indicating a position of the first detector moved by the traveling body. The processor outputs gas spatial distribution information based on the gas information from the first detector and the position information from the position information acquisition unit.

According to Aspect 6, in the gas detection system of Aspect 5, the processor outputs the gas spatial distribution information in association with map information.

According to Aspect 7, in the gas detection system of Aspect 5 or 6, the position information acquisition unit outputs the position information based on information received from a global position system.

According to Aspect 8, the gas detection system of any one of Aspects 5 to 7 further includes an image capturing device capable of outputting a captured image of surroundings of the traveling body, and the processor outputs the captured image associated with the position of the first detector.

## Claims

1. A gas detection device (500) comprising:
a first detector (100) configured to output gas information being information on a gas sucked from each of a plurality of inlets (21) arranged at different positions;
a second detector (200) configured to output object position information indicating a position of an object; and
a processor (300; 300a) configured to output, based on gas spatial distribution information based on the gas information from the first detector (100), the object position information from the second detector (200), and first relative position information indicating a position of each of the plurality of inlets relative to the object, second relative position information indicating gas spatial distribution relative to the position of the object.

2. The gas detection device (500) according to claim 1,
wherein the first relative position information is determined in advance.

3. The gas detection device (500) according to claim 1,
wherein the second detector (200) is configured to output inlet position information indicating the position of each of the plurality of inlets, and
wherein the processor (300; 300a) is configured to calculate the first relative position information based on the inlet position information.

4. The gas detection device (500) according to any one of claims 1 to 3,
wherein the second detector (200) includes at least one of an image capturing device (220) configured to output a captured image of the object and a distance-measuring device (220) configured to output distance information relative to the object.

5. A gas detection system (700) comprising:
a traveling body (150) including a detector (100b) configured to output gas information being information on inhaled gas;
a position information acquisition unit (200b) configured to output position information indicating a position of the first detector (100b) moved by the traveling body (150); and
a processor (300b) configured to output gas spatial distribution information based on the gas information from the first detector (100b) and the position information from the position information acquisition unit (200b).

6. The gas detection system (700) according to claim 5,
wherein the processor (300b) is configured to output the gas spatial distribution information in association with map information.

7. The gas detection system (700) according to claim 5 or 6,
wherein the position information acquisition unit (200b) is configured to output the position information based on information received from a global position system.

8. The gas detection system (700) according to any one of claims 5 to 7, further comprising an image capturing device (250) configured to output a captured image of surroundings of the traveling body (150),
wherein the processor (300b) is configured to output the captured image associated with the position of the first detector (100b).
